# EUROPEAN PATENT SPECIFICATION

(11) **EP 1 553 399 B1**
(45) Date of publication and mention of the grant of the patent: **10.10.2012**
(21) Application number: 04257407.9
(22) Date of filing: 30.11.2004
(51) Int. Cl.: G01N 21/35, A61B 5/00, G01N 33/48, G01J 3/18, A61B 5/1455, A61B 5/145

(54) **Body fluid components measuring apparatus and method**
Vorrichtung und Verfahren zur Messung von Bestandteilen einer Körperflüssigkeit
Dispositif et procédé pour la détermination de composants d'un fluide corporel

(30) Priority: 12.01.2004 KR 2004001961
(43) Date of publication of application: 13.07.2005
(73) Proprietor: Samsung Electronics Co., Ltd., Suwon-si, Gyeonggi-do, 443-742 (KR)
(72) Inventor: Hwang, In-duk, Suwon-si Gyeonggi-do (KR); Han, Wan-taek, Hwaseong-si Gyeonggi-do (KR); Jeon, Kye-jin, Suwon-si Gyeonggi-do (KR); Park, Kun-kook, Samsung Advanced Inst. of Techn., Yongin-si Gyeonggi-do (KR); Choe, Eun-young, Suwon-si Gyeonggi-do (KR); Hwang, Hyun-tai, Samsung Advanced Inst. of Techn., Yongin-si Gyeonggi-do (KR)
(74) Representative: Anderson, James Edward George

(56) References cited:
- EP-A- 0 075 171
- US-A- 4 882 492
- US-A- 5 348 003
- US-A- 5 365 066
- US-A- 5 676 141
- US-B1- 6 191 860
- PATENT ABSTRACTS OF JAPAN vol. 013, no. 101 (C-574), 9 March 1989 (1989-03-09) & JP 63 277040 A (HAMAMATSU PHOTONICS KK), 15 November 1988 (1988-11-15)
- PATENT ABSTRACTS OF JAPAN vol. 006, no. 192 (P-145), 30 September 1982 (1982-09-30) & JP 57 101743 A (MITSUBISHI ELECTRIC CORP), 24 June 1982 (1982-06-24)

## Description

The present invention relates to measuring a body component, and more specifically, to body component measuring apparatus and method using a scanning spectroscopic system.

In recent times, with large improvement of living environments and living conditions, people have had an increasing interest in health. As a result, development for home medical instruments capable of easily checking health at any time has been studied and new instruments have been developed rapidly.

In normal persons, their body fluids are organically circulated and adjusted, so that quantities of the body are kept within constant ranges. Examples of the body fluids include blood, urine, interstitial fluid, sweat, saliva, etc., and concentrations of the body fluids such as blood, urine (glucose, protein), etc. serve as very important variables indicating health. Further, concentrations of glucose, hemoglobin, bilirubin, cholesterol, albumin, creatinine, protein, urea, etc. are important measurement targets.

However, when a living body suffers from a certain disease, the compositions or quantities of body components may be varied, thereby resulting in a dangerous condition. For example, the concentration of blood glucose of a normal person is about 80 mg/dl before a meal and about 120 mg/dl after a meal. In order to keep the concentration of blood glucose constant, the living body allows pancreas to secrete a proper quantity of insulin before a meal or after a meal and allows liver and skeletal muscle cells to absorb the insulin.

However, in a case where the pancreas does not produce the proper quantity of insulin required for normally keeping the blood glucose due to a disease or other reasons, an excessive quantity of glucose may exist in blood. As a result, heart and liver troubles, arteriosclerosis, hypertension, cataract, retina hemorrhage, nerve damage, hearing loss, amblyopia, etc. may be caused, and in a severer case, death may be caused.

Before such an extreme result is caused, it is very important to measure variation in concentrations of in-vivo components. The methods of measuring concentrations of body components can be classified into an invasive method of directly taking parts of measurement target substances and measuring the concentrations and a non-invasive method of not taking the target substances and measuring the concentrations. However, since the invasive method has various problems, the methods of non-invasively measuring body components have been developed.

In conventionally measuring blood glucose, first, blood is taken from a living body and is allowed to react with a medical reagent. Then, the measurement is performed by using clinical analyzers or by quantifying discoloration of the test strip having reacted with the reagent. The taking of blood may cause large pains to the diabetic who are subjected to the measurement and may increase the possibility of inspection with diseases. Further, continuous monitoring thereof is difficult, so that it can be difficult to cope with emergencies. Furthermore, since expendable supplies should be used for the strip or reagent, large economical burdens are imposed on the users, and since the expendable supplies may cause environmental pollution, they must be processed.

Therefore, for the purpose of blood glucose adjustment of the diabetic or medical checkup of normal persons, development of techniques capable of measuring a blood glucose concentration without requiring strips or expendable supplies and without taking blood has been required. For this purpose, the methods of non-invasively measuring blood glucose have been actively studied, but commercialized instruments do not come to the market yet.

In most of the conventional spectroscopic methods used for measuring concentrations of body components, light components having visible ray and near-infrared ray wavelength bands are applied to a part of a biological tissue and the reflected or transmitted light components are detected, where the spectra are measured to estimate the concentrations. Specifically, a light source of a wavelength band most sensitive to the component to be measured and a reference light source of a wavelength band effectively canceling influence of interfering materials are required for estimating the concentration of a specific target component. Conventionally, since a light source such as a continuous wave lamp is used, there are disadvantages that the light intensity should be measured using an expensive array detector or that a plurality of light emitting diodes or laser diodes should be used for measuring a spectrum using a spectroscopic system and estimating the concentration.

Furthermore, in the conventional methods, when the body component to be measured has a very low concentration in blood and has a light scattering effect larger than a light absorbing effect, the detected signal is very weak, so that a method of amplifying the signal is necessary. Furthermore, since the organic substances in a living body flows continuously, the measuring time should be short for accurately measuring the component concentration. The average energy of the total light components to be applied to the human body must not exceed the limit value at which the human tissue can be damaged. Specifically, in the near-infrared area of 700 nm to 2,500 nm, since the absorption band of glucose is distributed widely and the absorption peak of glucose is relatively small compared with a large aqueous background spectrum, the signal-to-noise ratio is small, so that it is much difficult to accurately measure the concentration.

One of the methods of non-invasively measuring blood glucose is disclosed in U.S. Patent No. 5,086,229, which is entitled "NON-INVASIVE MEASUREMENT OF BLOOD GLUCOSE" and allowed to Rogental et al on February 2, 1992. In this method, light components having a wavelength of 600 nm to 1,100 nm are used and a plurality of different light emitting diodes should be used for generating the light components having the above wavelength, so that energy difference of in-vivo reflected or transmitted light components can be compared and analyzed to estimate the glucose concentration. However, since a large number of light emitting diodes should be combined in order to obtain sufficient signals from the disclosed absorption wavelength of glucose, there is a problem that it is difficult to align the light emitting diodes having different wavelengths. Furthermore, since the light emitting diodes having different wavelengths should be combined, the light components do not pass through the same position during application of the light components, so that it is difficult to accurately measure the concentration.

In U.S. Patent No. 5,222,495, which is entitled "NON-INVASIVE BLOOD ANALYSIS BY NEAR INFRARED ABSORPTION MEASUREMENT USING TWO CLOSELY SPACED WAVELENGTH" and allowed to Clarke et al on Jun 29, 1993, adjacent wavelengths are used in order to prevent various scattering phenomena in a biological body. That is, a light component having a wavelength of 1,600 nm is used as a signal light and a light component having a wavelength of 1,630 nm to 1,660 nm, which is larger than the signal wavelength but has a smaller wavelength difference, is used as a reference light component. By using this method, the spectrum has ever measured from a prepared sample, but has never measured in vivo.

U.S. Patent No. 6,191,860, which is entitled "OPTICAL SHUTTER, SPECTROMETER AND METHOD FOR SPECTRALANALYSIS" AND ALLOWED TO Klinger et al on February 20, 2001, discloses an optical shutter arrangement comprising a plurality of spatially distributed optical shutters provided with optical filters having respective different wavelength pass bands. The light passing through one or more of the shutters is focused using a lens. This U.S. patent also discloses the use of diffraction gratings in spectrometry, but only in connection with background art.

According to aspects of the present invention, there are provided the apparatus of claim 1 and the method of claim 10.

The present invention thus provides body component measuring apparatus and method, which can improve estimation ability of glucose concentration by analyzing two light components having two wavelength bands of 1,600 nm and 2,200 nm, which are absorbed by glucose, by using a scanning spectroscopic system.

The above and other features and advantages of the present invention will become more apparent from detailed description of exemplary embodiments thereof with reference to the attached drawings in which:
FIG. 1 is a diagram schematically illustrating a body component measuring apparatus comprising a scanning spectroscopic system according to an exemplary embodiment of the present invention; and
FIG. 2 is a diagram illustrating control of a shutter and an amplifier gain for the purpose of correcting a baseline according to an exemplary embodiment of the present invention.

Hereinafter, the present invention will be described in detail by explaining exemplary embodiments of the invention with reference to the attached drawings.

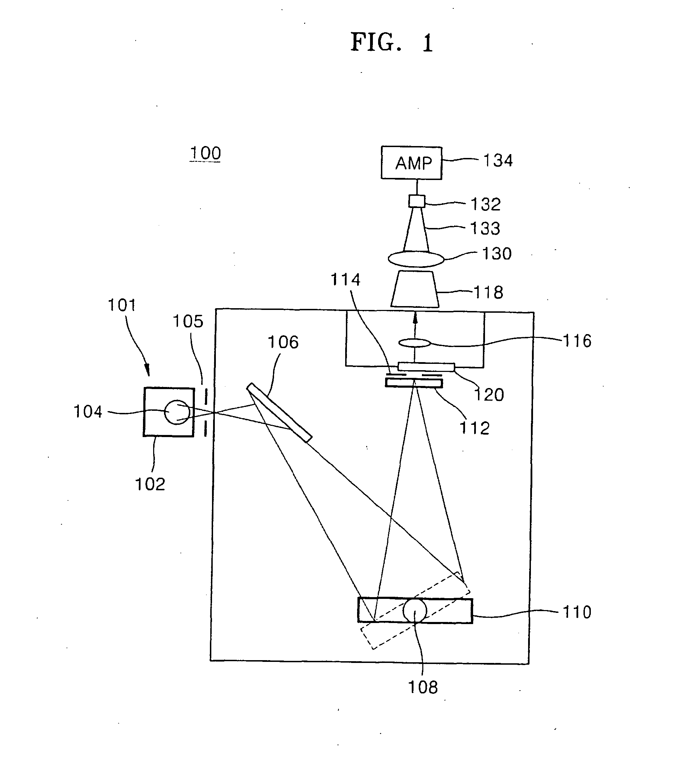

FIG. 1 is a diagram schematically illustrating a body component measuring apparatus comprising a scanning spectroscopic system according to an exemplary embodiment of the present invention.

A body component measuring apparatus 100 according to an exemplary embodiment of the present invention comprises a light source unit 101 having a light source 104 such as a halogen lamp and a power supply in a housing 102, first and second slits 105 and 114, a reflecting mirror 106 reflecting the light emitted from the light source 104, a diffraction grating 110 dispersing the light reflected from the reflecting mirror 106, a high pass filter 112 provided between the diffraction grating 110 and the second slit 114, a cylindrical lens 116, a light delivery unit 118, a shutter 120, a signal light delivery unit 133 guiding the light reflected from or passing through a measuring portion 130 into a detector 132, the detector 132, and an amplifier 134.

According to an exemplary embodiment of the present invention, the high pass filter 112 is provided between the second slit 114 positioned at the outlet of a spectroscopic system and the diffraction grating 110, and it is preferable that the high pass filter 112 is fixed right below the second slit 114.

The body component measuring apparatus 100 according to an exemplary embodiment of the present invention may further comprise a digital signal processing unit, a concentration estimating unit, and an analog-digital converting unit.

Now, operation of the body component measuring apparatus 100 according to an exemplary embodiment of the present invention will be described.

First, the light emitted from the light source 104 such as a halogen lamp passes through the first slit 105 provided at the inlet of the spectroscopic system and then is incident on the reflecting mirror 106. The incident light is incident on the diffraction grating 110, which is a light dispersing element, through the reflecting mirror 106. The diffraction grating 110 is coupled to a motor shaft 108 of a direct current (DC) motor and is rotated by means of a driving unit of the motor. According to an exemplary embodiment of the present invention, the diffraction grating 110 is formed out of a concave grating.

The light dispersed by the diffraction grating 110 passes through the second slit 114 provided at the outlet of the spectroscopic system and is focused on the light guide unit 118 through a cylindrical lens 116.

The light passing through the light guide unit 118 is incident on a sample cuvette retaining a body component or the measuring portion 130, which is a part of a biological tissue such as a finger web, or an ear lobe, and then measured. According to an exemplary embodiment of the present invention, since the refractive indexes between the surface of the biological tissue and an interface at which the light meets the surface of the biological tissue are different, it is possible to enhance the signal-to-noise ratio by providing a medium for matching the refractive indexes therebetween.

The signal light reflected or transmitted from the measuring portion 130 is sent to the detector 132 through the signal light delivery unit 133 having a lens, or a mirror, or combination thereof.

The analog signal detected by the detector 132 is amplified by the amplifier 134.

Although not shown in the drawings, the body component measuring apparatus 100 according to an exemplary embodiment of the present invention further comprises an analog-digital converter, a digital signal processing unit, a concentration estimating unit, and a microprocessor.

The amplified analog signal is converted into a digital signal, which can be processed by a microcomputer, through the analog-digital converter. Then, the signal output from the analog-digital converter is processed and analyzed by the digital signal processing unit, and the body component is estimated by the concentration estimating unit having a built-in algorithm for estimating concentrations of the body components to be measured, on the basis of data output from the digital signal processing unit. The microprocessor controls all the processes such as emitting and receiving the light, estimating the concentration, and others.

The concentration estimating unit prepares an estimation model equation capable of estimating an accurate concentration of the body component by comparing and analyzing the degrees of absorption by the wavelengths used for measurement, thereby calculating the concentration of the body component to be measured. Various known analyzing methods can be used for the statistical analysis for estimating the concentration.

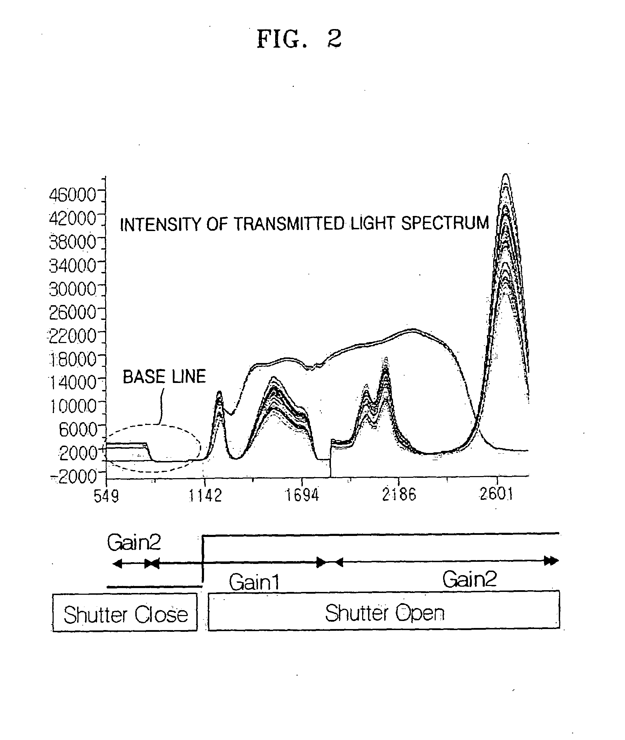

FIG. 2 is a diagram illustrating control of the shutter and the amplifier gain for the purpose of correcting a baseline according to an exemplary embodiment of the present invention. A portion corresponding to the baseline is shown in FIG. 2.

For the purpose of calibrating the wavelengths, a polystyrene plate is provided between the cylindrical lens 116 and the second slit 114 under control of the DC motor, and the measured spectrum is calculated by means of the built-in software, thereby calculating accurate wavelengths.

The polystyrene plate according to an exemplary embodiment of the present invention has an absorption peak at a specific wavelength such as 1,680.90 nm, 2,166.72 nm, 2,306.10nm, etc. Preferably, the polystyrene plate according to an exemplary embodiment of the present invention is formed to be 1 mm thick.

In an exemplary embodiment of the present invention, glucose, which is one of body components, is exemplified, and in order to acquire a more intensive signal at 2,200 nm, which is an absorption band of glucose, the amplifier gain is controlled to be different from that of the wavelength band of 1,600 nm. The operation is as follows.

The actual absorbance is calculated by first measuring the transmitted light with a reference gain in the absence of a biological body and then measuring the transmitted light in the presence of the biological body. During the measurement, the shutter 120 is positioned between the cylindrical lens 116 and the second slit 114 at a predetermined time interval under control of the DC motor

First, the shutter 120 intercepting light is closed initially, and is opened at the wavelength of about 1,200 nm while being moved from a small wavelength side to a large wavelength side with rotation of the diffraction grating 110. Since intensity of light which the living body transmits is still smaller at the wavelength band of 2,200 nm than that at the wavelength band of 1,600 nm, the amplifier gain should be controlled to be still larger at the wavelength band of 2,200 nm than at the wavelength band of 1,600 nm, thereby more accurately estimating the concentration of blood glucose.

Therefore, since the amplifier gain is changed during one scanning, that is, one rotation of the diffraction grating 110, variation of a baseline due to change of the amplifier gain should be corrected. Signals corresponding to a first gain at the wavelength band of 1,600 nm and a second gain at the wavelength band of 2,200 nm are measured in synchronism with the opening and closing of the shutter 120, the baseline is corrected, and then the final absorbance is calculated using this corrected value. To estimate the concentration of body components, various absorbance data measured according to a concentration change of a specific body component are used. In particular, the concentration of a target body component is estimated using a conventional statistical analysis method.

The body component measuring apparatus 100 according to an exemplary embodiment of the present invention can be effectively used in various similar application systems as well as in measuring the body component.

According to an exemplary embodiment of the present invention, light having a wavelength of 1,200 nm to 2,400 nm is used for measuring the concentration of blood glucose.

Further, the tungsten halogen lamp generating continuous spectrums at the area ranging from the visible ray to the near-infrared ray is used as the light source, and the high pass filter transmitting only the light components having a wavelength band of 1,200 nm or more is provided right below the second slit 114 in the spectroscopic system for the purpose of reducing temperature rise at the measuring portion and not using an order sorting filter.

Furthermore, according to an exemplary embodiment of the present invention, the detector 132 determines the baseline value to be used for correcting the signals in accordance with one or more different values of the amplifier gain, obtained by controlling that the shutter 120 intercepts the light components having a wavelength of 1,200 nm or less.

Furthermore, according to the exemplary embodiment of the present invention, the calibration of wavelength is performed by using the structure in which the high pass filter and the polystyrene film are coupled in series. After completing the calibration of wavelength, the polystyrene film is removed and only the high pass filter 122 is put in the optical path.

Furthermore, it is preferable that the scanning speed of the diffraction grating 110 provided in the spectroscopic system is set to 2 scan/sec. This is because it is necessary that the measuring time should be reduced as small as possible to minimize influence of continuous circulation of in-vivo organic substances.

Furthermore, according to an exemplary embodiment of the present invention, by allowing the light components incident to the measuring portion to have specific wavelength bands through scanning the diffraction grating, it is possible to invasively or non-invasively measure the concentration of the body component.

In an exemplary embodiment of the present invention, in order to measure the concentration of glucose, a wide-band spectrum including a wavelength band to be largely absorbed by glucose and a wavelength band to be small absorbed by glucose is obtained by rotating the diffraction grating and thus dispersing the light. First, the dispersed light components is applied to the biological tissue. And then by statistically analyzing the absorption differences of signal lights reflected or transmitted from the biological tissue, it is possible to accurately estimate the concentration of blood glucose.

Furthermore, in an exemplary embodiment of the present invention, since the intensity of the light component having a wavelength of 2,200 nm, which is an absorption wavelength band of glucose, is several tens times smaller than the intensity of the light component having a wavelength of 1,600 nm after the light components passes through the biological tissue, the amplifier gain has different values between the two wavelength bands, so that it is possible to enhance the signal-to-noise ratio and to enhance the resolution.

As described above, according to the present invention, by applying the different amplifier gain values to two specific wavelength bands, it is possible to enhance the accuracy for estimating the concentration of a body component.

Further, according to the exemplary embodiment of the present invention, by scanning the diffraction grating at high speed, it is possible to reduce the measuring time. In addition, since only the light components required for estimating the concentration of a body component is input to the biological tissue, it is possible to minimize the variation of measurement due to the temperature rise of the biological tissue.

Furthermore, according to the exemplary embodiment of the present invention, it is possible to invasively or non-invasively measure the concentration of a body component, and it is also possible to provide a simple body component measuring apparatus with decreased size and weight.

## Claims

1. A body component measuring apparatus for measuring a body component in a specific portion of a living body, the apparatus comprising:
a light source (104) for emitting light;
a movable diffraction grating (110) for dispersing the light emitted from the light source (104) into light components having at least first and second wavelength bands different from each other;
a shutter arrangement (120) for controlling transmission of the light components having the respective plurality of wavelength bands;
a lens (116) for focusing the light components passing through the shutter arrangement (120) on the specific portion according to an opening or closing of the shutter arrangement (120);
a detector (132) for detecting the light components passing through the specific portion;
an amplifier (134) for amplifying signals corresponding to the light components detected by the detector (132);
an analog-digital converter converting the detected signals into digital signals;
a digital signal processing unit;
a micro processor; and
a concentration estimating unit configured to use the amplified signals corresponding to the at least first and second wavelength bands of the light components to estimate a concentration of the body component,wherein the apparatus is **characterized in that** the diffraction grating (110) directs each of the light components of the at least first and second wavelength bands through a same shutter of the shutter arrangement (120);
wherein the amplifier (134) provides a first amplifier gain for a signal corresponding to the first wavelength band and a second amplifier gain for a signal corresponding to the second wavelength band, and
wherein the detector (132) determines the base line value to be used for correcting the signals in accordance with one or more different values of the amplifier gain,
wherein the analog-digital converter is adapted to convert said corrected signals into digital signals, the apparatus (100) is adapted to calculate a final absorbance based on said digital signals, and wherein
the concentration estimating unit is adapted to calculate the concentration of the body component based on said final absorbance.

2. The apparatus according to claim 1, wherein the body component is glucose, and the first wavelength band is a wavelength band of about 1,600 nm and the second wavelength band is a wavelength band of about 2,200 nm.

3. The apparatus according to claim 2, wherein the apparatus is arranged so that, when the shutter is opened, the second amplifier gain for the light component having the second wavelength band of about 2,200 nm is larger than the first amplifier gain for the light component having the first wavelength band of about 1,600 nm.

4. The apparatus according to any of claims 1 to 3, wherein the apparatus is arranged such that, when the shutter is opened, amplifier gains differ according to the wavelength bands of the light components.

5. The apparatus according to any preceding claim, further comprising:
an optical system (105, 106) for inputting the light to the diffraction grating (110);
a high pass filter (112) arranged between the diffraction grating (110) and the shutter (120);
a light guide unit (118) arranged between the lens (116) and the specific portion of the living body; and
a signal light delivery unit (133) for delivering the light from the specific portion to the detector (132).

6. The apparatus according to claim 5, wherein the high pass filter (112) is arranged to transmit a light component having a wavelength of 1,200 nm or more.

7. The apparatus according to claim 5 or 6, further comprising a polystyrene film for coupling in series to the high pass filter (112) for the purpose of calibration of the wavelengths of the light components.

8. The apparatus according to claim 7, wherein the polystyrene film is removable for user of the apparatus after performing the calibration.

9. The apparatus according to any preceding claim, further comprising a motor (108) for rotating the diffraction grating (110) at high speed, so that movement of the diffraction grating (110) is rotary movement.

10. A body component measuring method for measuring a body component in a specific portion of a living body, the method comprising:
generating light components having at least first and second wavelength
bands different from each other by using a movable diffraction grating (110) to disperse light emitted from a light source (104);
controlling transmission of the light components having the respective plurality of wavelength bands using a shutter arrangement (120);
focusing the light components passing through the shutter arrangement (120) on the specific portion using a lens (116), according to an opening or closing of the shutter arrangement (120);
detecting the light components passing through the specific portion using a detector (132);
amplifying a signal corresponding to the first wavelength band detected by the detector (132) with a first amplifier gain and a signal corresponding to the second wavelength band detected by the detector (132) with a second amplifier gain using an amplifier (134); converting the signal into a digital signal through an analog-digital converter; and
estimating the body component by using the detected light components,
wherein the method is **characterised in that** the diffraction grating (110) directs each of the light components having the at least first and second wavelength bands through a same shutter of the shutter arrangement (120), and wherein, when the shutter is opened, amplifier gains applied to the detected light components differ according to the wavelength bands of the light components, and
wherein, after the variation of a base line due to change of the amplifier gains is corrected, the concentration of the body component is estimated from a final absorbance which is calculated based on the data output from the digital signal processing unit which has been corrected for said variation in the baseline.

11. The method according to claim 10, wherein the method is a method for measuring glucose, the first wavelength band is a wavelength band of about 1,600 nm and the second wavelength band is a wavelength band of about 2,200 nm.

12. The method according to claim 11, wherein, when the shutter is opened, the second amplifier gain for the light component having the wavelength band of about 2,200 nm is larger than that the first amplifier gain for the light component having the wavelength of about 1,600 nm.

13. The method according to any of claims 10 to 12, wherein the step of generating light components further comprises calibrating the wavelengths of the light components using a polystyrene film coupled in series to a high pass filter (112).

14. The method according to claim 13, wherein the polystyrene film is removed after calibrating the wavelengths.

15. The method according to any of claims 10 to 14, wherein the generating of the light components is performed by scanning the diffraction grating (110) with a motor (108) at high speed.

## Patentansprüche

1. Körperbestandteil-Messvorrichtung zum Messen eines Körperbestandteils in einem bestimmten Abschnitt eines lebenden Körpers, wobei die Vorrichtung Folgendes umfasst:
eine Lichtquelle (104) zum Ausstrahlen von Licht;
ein bewegbares Beugungsgitter (110) zum Streuen des von der Lichtquelle (104) ausgestrahlten Lichts in Lichtkomponenten mit mindestens einem ersten und einem zweiten, voneinander verschiedenen Wellenlängenband;
eine Verschlussordnung (120) zum Regeln der Übertragung der Lichtkomponenten mit den jeweiligen mehreren Wellenlängenbändern;
eine Linse (116) zum Fokussieren der durch die Verschlussanordnung (120) gelangenden Lichtkomponenten auf den bestimmten Abschnitt gemäß einem Öffnen oder Schließen der Verschlussanordnung (120);
einen Detektor (132) zum Erkennen der durch den bestimmten Abschnitt gelangenden Lichtkomponenten;
einen Verstärker (134) zum Verstärken von Signalen, die den von dem Detektor (132) erkannten Lichtkomponenten entsprechen;
einen Analog-Digital-Wandler, der die erkannten Signale in Digitalsignale wandelt;
eine Digitalsignal-Verarbeitungseinheit;
einen Mikroprozessor; und
eine Konzentrationsabschätzungseinheit, die dazu ausgebildet ist, die dem mindestens ersten und zweiten Wellenlängenband der Lichtkomponenten entsprechenden verstärkten Signale zu verwenden, um eine Konzentration des Körperbestandteils abzuschätzen, wobei die Vorrichtung **dadurch gekennzeichnet ist, dass** das Beugungsgitter (110) jede der Lichtkomponenten des mindestens ersten und zweiten Wellenlängenbands durch den selben Verschluss der Verschlussanordnung (120) leitet;
wobei der Verstärker (134) eine erste Verstärkerverstärkung für ein dem ersten Wellenlängenband entsprechendes Signal und eine zweite Verstärkerverstärkung für ein dem zweiten Wellenlängenband entsprechendes Signal bereitstellt; und
wobei der Detektor (132) den zum Korrigieren der Signale gemäß einem oder mehreren verschiedenen Werten der Verstärkerverstärkung zu verwendenden Grundlinienwert bestimmt,
wobei der Analog-Digital-Wandler dazu angepasst ist, die genannte korrigierten Signale in Digitalsignale zu wandeln, die Vorrichtung (100) dazu angepasst ist, eine endgültige Absorbanz basierend auf den genannten Digitalsignalen zu berechnen, und wobei
die Konzentrationsabschätzungseinheit dazu angepasst ist, die Konzentration des Körperbestandteils basierend auf der genannten endgültigen Absorbanz zu berechnen.

2. Vorrichtung nach Anspruch 1, wobei es sich bei dem Körperbestandteil um Glukose handelt und das erste Wellenlängenband ein Wellenlängenband von ungefähr 1600 nm ist und das zweite Wellenlängenband ein Wellenlängenband von ungefähr 2200 nm ist.

3. Vorrichtung nach Anspruch 2, wobei die Vorrichtung derart angeordnet ist, dass wenn der Verschluss geöffnet ist, die zweite Verstärkerverstärkung für die Lichtkomponente mit dem zweiten Wellenlängenband von ungefähr 2200 nm größer ist als die erste Verstärkerverstärkung für die Lichtkomponente mit dem ersten Wellenlängenband von ungefähr 1600 nm.

4. Vorrichtung nach einem der Ansprüche 1 bis 3, wobei die Vorrichtung derart angeordnet ist, dass wenn der Verschluss geöffnet ist, Verstärkerverstärkungen gemäß den Wellenlängenbändern der Lichtkomponenten verschieden sind.

5. Vorrichtung nach einem der vorangehenden Ansprüche, die weiter Folgendes umfasst:
ein optisches System (105, 106) zum Eingeben des Lichts an das Beugungsgitter (110);
einen Hochpassfilter (112), der zwischen dem Beugungsgitter (110) und dem Verschluss (120) angeordnet ist;
eine Lichtleitereinheit (118), die zwischen der Linse (116) und dem bestimmen Abschnitt des lebenden Körpers angeordnet ist; und
eine Signallicht-Zuführeinheit (133) zum Zuführen des Lichts von dem bestimmten Abschnitt zu dem Detektor (132).

6. Vorrichtung nach Anspruch 5, wobei der Hochpassfilter (112) dazu angeordnet ist, eine Lichtkomponente mit einer Wellenlänge von 1200 nm oder mehr zu übertragen.

7. Vorrichtung nach Anspruch 5 oder 6, weiter umfassend einen Polystyrolfilm zum Koppeln in Reihe an den Hochpassfilter (112) für den Zweck der Kalibration der Wellenlängen der Lichtkomponenten.

8. Vorrichtung nach Anspruch 7, wobei der Polystyrolfilm nach dem Ausführen der Kalibration für den Benutzer der Vorrichtung entfernbar ist.

9. Vorrichtung nach einem der vorangehenden Ansprüche, weiter umfassend einen Motor (108) zum Drehen des Beugungsgitters (110) mit hoher Geschwindigkeit, so dass es sich bei der Bewegung des Beugungsgitters (110) um Drehbewegung handelt.

10. Körperbestandteil-Messverfahren zum Messen eines Körperbestandteils in einem bestimmten Abschnitt eines lebenden Körpers, wobei das Verfahren Folgendes umfasst:
Erzeugen von Lichtkomponenten mit mindestens einem ersten und einem zweiten voneinander verschiedenen Wellenlängenband durch Verwenden eines bewegbaren Beugungsgitters (110) zum Streuen von von einer Lichtquelle (104) ausgestrahltem Licht;
Regeln der Übertragung der Lichtkomponenten mit den jeweiligen mehreren Wellenlängenbändern unter Verwendung einer Verschlussanordnung (120);
Fokussieren der durch die Verschlussanordnung (120) gelangenden Lichtkomponenten auf den bestimmten Abschnitt unter Verwendung einer Linse (116), gemäß einem Öffnen oder Schließen der Verschlussanordnung (120);
Erkennen der durch den bestimmten Abschnitt gelangenden Lichtkomponenten unter Verwendung eines Detektors (132);
Verstärken eines dem ersten von dem Detektor (132) erkannten Wellenlängenband entsprechenden Signals mit einer ersten Verstärkerverstärkung und eines dem zweiten von dem Detektor (132) erkannten Wellenlängenband entsprechenden Signals mit einer zweiten Verstärkerverstärkung unter Verwendung eines Verstärkers (134);
Wandeln des Signals in ein Digitalsignal über einen Analog-Digital-Wandler; und
Abschätzen des Körperbestandteils durch Verwenden der erkannten Lichtkomponenten,
wobei das Verfahren **dadurch gekennzeichnet ist, dass** das Beugungsgitter (110) jede der Lichtkomponenten mit dem mindestens ersten und zweiten Wellenlängenband durch den selben Verschluss der Verschlussanordnung (120) leitet und wobei wenn der Verschluss geöffnet ist, auf die erkannten Lichtkomponenten angewandte Verstärkerverstärkungen gemäß den Wellenlängenbändern der Lichtkomponenten verschieden sind, und
wobei, nachdem die Abweichung einer Grundlinie infolge Änderung der Verstärkerverstärkungen korrigiert ist, die Konzentration des Körperbestandteils aus einer endgültigen Absorbanz abgeschätzt wird, die basierend auf dem Datenausgang von der Digitalsignalverarbeitungseinheit berechnet wird, der um die genannte Abweichung der Grundlinie korrigiert wurde.

11. Verfahren nach Anspruch 10, wobei es sich bei dem Verfahren um ein Verfahren zum Messen von Glukose handelt, das erste Wellenlängenband ein Wellenlängenband von ungefähr 1600 nm ist und das zweite Wellenlängenband ein Wellenlängenband von ungefähr 2200 nm ist.

12. Verfahren Anspruch 11, wobei wenn der Verschluss geöffnet ist, die zweite Verstärkerverstärkung für die Lichtkomponente mit dem Wellenlängenband von ungefähr 2200 nm größer ist als die erste Verstärkerverstärkung für die Lichtkomponente mit der Wellenlänge von ungefähr 1600 nm.

13. Verfahren nach einem der Ansprüche 10 bis 12, wobei der Schritt des Erzeugens von Lichtkomponenten weiter das Kalibrieren der Wellenlängen der Lichtkomponenten unter Verwendung eines in Reihe an einen Hochpassfilter (112) gekoppelten Polystyrolfilms umfasst.

14. Verfahren nach Anspruch 13, wobei der Polystyrolfilm nach dem Kalibrieren der Wellenlängen entfernt wird.

15. Verfahren nach einem der Ansprüche 10 bis 14, wobei das Erzeugen der Lichtkomponenten durch Abtasten des Beugungsgitters (110) mit einem Motor (108) bei hoher Geschwindigkeit ausgeführt wird.

## Revendications

1. Appareil de mesure de composant de corps pour mesurer un composant de corps dans une portion spécifique d'un corps vivant, l'appareil comprenant :
une source de lumière (104) pour émettre de la lumière ;
un réseau de diffraction mobile (110) pour disperser la lumière émise par la source de lumière (104) en composants lumineux ayant au moins des première et seconde bandes de longueur d'onde différentes l'une de l'autre ;
un agencement d'obturateur (120) pour commander la transmission des composants lumineux ayant la pluralité respective de bandes de longueur d'onde ;
une lentille (116) pour focaliser les composants lumineux passant à travers l'agencement d'obturateur (210) sur la portion spécifique selon une ouverture ou une fermeture de l'agencement d'obturateur (120) ;
un détecteur (132) pour détecter les composants lumineux passant à travers la portion spécifique ;
un amplificateur (134) pour amplifier des signaux correspondant aux composants lumineux détectés par le détecteur (132) ;
un convertisseur analogique/numérique convertissant les signaux détectés en signaux numériques ;
une unité de traitement de signal numérique ;
un microprocesseur ; et
une unité d'estimation de concentration configurée pour utiliser les signaux amplifiés correspondant aux au moins première et seconde bandes de longueur d'onde des composants lumineux pour estimer une concentration du composant de corps, dans lequel l'appareil est **caractérisé en ce que** le réseau de diffraction (110) dirige chacun des composants lumineux des au moins première et seconde bandes de longueur d'onde à travers un même obturateur de l'agencement d'obturateur (120) ;
dans lequel l'amplificateur (134) fournit un premier gain d'amplificateur pour un signal correspondant à la première bande de longueur d'onde et un second gain d'amplificateur pour un signal correspondant à la seconde bande de longueur d'onde, et
dans lequel le détecteur (132) détermine la valeur de ligne de base devant être utilisée pour corriger les signaux en conformité avec une ou plusieurs valeurs différentes du gain d'amplificateur,
dans lequel le convertisseur analogique-numérique est adapté pour convertir lesdits signaux corrigés en signaux numériques, l'appareil (100) est adapté pour calculer une absorbance finale en se basant sur lesdits signaux numériques, et dans lequel
l'unité d'estimation de concentration est adaptée pour calculer la concentration du composant de corps en se basant sur ladite absorbance finale.

2. Appareil selon la revendication 1, dans lequel le composant de corps est le glucose, et la première bande de longueur d'onde est une bande de longueur d'onde d'environ 1 600 nm et la seconde bande de longueur d'onde est une bande de longueur d'onde d'environ 2 200 nm.

3. Appareil selon la revendication 2, dans lequel l'appareil est agencé de sorte que, lorsque l'obturateur est ouvert, le second gain d'amplificateur pour le composant lumineux ayant la seconde bande de longueur d'onde d'environ 2 200 nm est plus grand que le premier gain d'amplificateur pour le composant lumineux ayant la première bande de longueur d'onde d'environ 1 600 nm.

4. Appareil selon l'une quelconque des revendications 1 à 3, dans lequel l'appareil est agencé de sorte que, lorsque l'obturateur est ouvert, le gain d'amplificateur diffère selon les bandes de longueur d'onde des composants lumineux.

5. Appareil selon l'une quelconque des revendications précédentes, comprenant en outre :
un système optique (105, 106) pour introduire la lumière vers le réseau de diffraction (110) ;
un filtre passe-haut (112) agencé entre le réseau de diffraction (110) et l'obturateur (120) ;
une unité de guide de lumière (118) agencée entre la lentille (116) et la portion spécifique du corps vivant ; et
une unité d'administration de lumière de signal (133) pour administrer la lumière en provenance de la portion spécifique au détecteur (132).

6. Appareil selon la revendication 5, dans lequel le filtre passe-haut (112) est agencé pour transmettre un composant lumineux ayant une longueur d'onde de 1 200 nm ou plus.

7. Appareil selon la revendication 5 ou 6, comprenant en outre un film en polystyrène pour couplage en série au filtre passe-haut (112) dans le but d'étalonner les longueurs d'onde des composants lumineux.

8. Appareil selon la revendication 7, dans lequel le film en polystyrène est amovible pour l'utilisateur de l'appareil après réalisation de l'étalonnage.

9. Appareil selon l'une quelconque des revendications précédentes, comprenant en outre un moteur (108) pour mettre en rotation le réseau de diffraction (110) à grande vitesse, de sorte qu'un mouvement du réseau de diffraction (110) est un mouvement rotatif.

10. Procédé de mesure de composant de corps pour mesurer un composant de corps dans une portion spécifique d'un corps vivant, le procédé comprenant les étapes consistant à:
générer des composants lumineux ayant au moins des première et seconde bandes de longueur d'onde différentes l'une de l'autre en utilisant un réseau de diffraction mobile (110) pour disperser la lumière émise par une source de lumière (104) ;
commander la transmission des composants lumineux ayant la pluralité respective de bandes de longueur d'onde en utilisant un agencement d'obturateur (120) ;
focaliser les composants lumineux passant à travers l'agencement d'obturateur (120) sur la portion spécifique en utilisant une lentille (116) selon une ouverture ou une fermeture de l'agencement d'obturateur (120) ;
détecter les composants lumineux passant à travers la portion spécifique en utilisant un détecteur (132) ;
amplifier un signal correspondant à la première bande de longueur d'onde détecté par le détecteur (132) avec un premier gain d'amplificateur et un signal correspondant à la seconde bande de longueur d'onde détecté par le détecteur (132) avec un second gain d'amplificateur utilisant un amplificateur (134) ; convertir le signal en un signal numérique via un convertisseur analogique-numérique ; et
estimer le composant de corps en utilisant les composants lumineux détectés,
dans lequel le procédé est **caractérisé en ce que** le réseau de diffraction (110) dirige chacun des composants lumineux ayant les au moins première et seconde bandes de longueur d'onde à travers un même obturateur de l'agencement d'obturateur (120), et dans lequel, lorsque l'obturateur est ouvert, les gains d'amplificateur appliqués aux composants de lumière détectés diffèrent selon les bandes de longueur d'onde des composants lumineux, et
dans lequel, après que la variation d'une ligne de base due à un changement des gains d'amplificateur est corrigée, la concentration du composant de corps est estimée à partir d'une absorbance finale qui est calculée en se basant sur les données produites en sortie par l'unité de traitement de signal numérique dont ladite variation dans la ligne de base a été corrigée.

11. Procédé selon la revendication 10, dans lequel le procédé est un procédé de mesure du glucose, la première bande de longueur d'onde est une bande de longueur d'onde d'environ 1 600 nm et la seconde bande de longueur d'onde est une bande de longueur d'onde d'environ 2 200 nm.

12. Procédé selon la revendication 11, dans lequel, lorsque l'obturateur est ouvert, le second gain d'amplificateur pour le composant lumineux ayant la bande de longueur d'onde d'environ 2 200 nm est plus grand que le premier gain d'amplificateur pour le composant lumineux ayant la longueur d'onde d'environ 1 600 nm.

13. Procédé selon l'une quelconque des revendications 10 à 12, dans lequel l'étape de génération de composants lumineux comprend en outre l'étalonnage des longueurs d'onde des composants lumineux en utilisant un film en polystyrène couplé en série à un filtre passe-haut (112).

14. Procédé selon la revendication 13, dans lequel le film en polystyrène est retiré après étalonnage des longueurs d'onde.

15. Procédé selon l'une quelconque des revendications 10 à 14, dans lequel la génération des composants lumineux est réalisée en balayant le réseau de diffraction (110) avec un moteur (108) à grande vitesse.
